# EUROPÄISCHE PATENTSCHRIFT

(11) **EP 2 916 723 B1**
(45) Veröffentlichungstag und Bekanntmachung des Hinweises auf die Patenterteilung: **29.07.2020**
(21) Anmeldenummer: 13789784.9
(22) Anmeldetag: 12.11.2013
(51) Int. Cl.: A61B 5/021, A61B 17/132, A61B 17/135

(54) **STEUEREINRICHTUNG UND STEUERSYSTEM FÜR EIN BLUTSPERREGERÄT**
CONTROL APPARATUS AND CONTROL SYSTEM FOR A TOURNIQUET DEVICE
DISPOSITIF ET SYSTÈME DE COMMANDE POUR UN APPAREIL DE RÉALISATION DE GARROT

(30) Priorität: 12.11.2012 DE 102012110827
(43) Veröffentlichungstag der Anmeldung: 16.09.2015
(73) Patentinhaber: Ulrich GmbH & Co. KG, 89081 Ulm (DE)
(72) Erfinder: KRAHWINKEL, Martin, 89081 Ulm (DE); HÄNSLER, Armin, 88471 Laupheim (DE); ERDMANN, Sven, 89081 Ulm (DE)
(74) Vertreter: Charrier Rapp & Liebau
(86) Internationale Anmeldenummer: PCT/EP2013/073561
(87) Internationale Veröffentlichungsnummer: WO 2014/072520

(56) Entgegenhaltungen:
- EP-A2- 0 122 123
- WO-A1-93/06782
- WO-A1-94/22364
- DE-A1- 4 008 711
- US-A1- 2006 293 601
- US-A1- 2011 046 494

## Beschreibung

Die Erfindung betrifft eine Steuereinrichtung für ein Blutsperregerät nach dem Oberbegriff des Anspruchs 1, sowie die Verwendung einer Steuereinrichtung zur Steuerung eins Blutsperregeräts.

Blutsperregeräte werden zur temporären Regulierung des arteriellen Blutflusses in den oberen und unteren Extremitäten des menschlichen oder tierischen Körpers verwendet. Bekannte Blutsperreeinrichtungen umfassen ein Blutsperregerät und eine daran anschließbare Kompressionsmanschette, welche an einer Extremität eines Patienten angelegt und anschließend aufgeblasen wird, um den arteriellen Blutfluss in der Extremität zu reduzieren, ggf. bis hin zu einer absoluten Blutsperre. Dauer und Höhe des durch die Kompressionsmanschette auf die Extremität ausgeübten Drucks werden dabei nach Vorgaben des behandelnden Arztes eingestellt. Typische Einsatzgebiete solcher Blutsperreeinrichtungen liegen im Bereich der chirurgischen und orthopädischen Operationen, jedoch auch in der Anästhesie (IVRA, "Intravenous Regional Anesthesia").

Die bekannten Blutsperregeräte weisen eine Aufblaseinrichtung zum Aufblasen der an das Blutsperregerät angeschlossenen Kompressionsmanschette sowie eine Steuereinrichtung zur Steuerung der Aufblaseinrichtung auf. Damit eine Blutsperreeinrichtung zur Erzeugung von Blutsperren an verschiedenen Extremitäten (bspw. am Oberarm, am Unterarm, am Oberschenkel oder am Unterschenkel) und auch an verschiedenen Patienten verwendet werden kann, sind in der Regel Kompressionsmanschetten unterschiedlicher Größe an ein Blutsperregerät anschließbar. Je nach Umfang der Extremität wird eine passende Kompressionsmanschette ausgewählt, an der Extremität angelegt und mittels der Aufblaseinrichtung des Blutsperregeräts auf einen vorgegebenen Druck und über eine vorgegebene Blutsperredauer aufgeblasen, um den Blutfluss in der Extremität zu begrenzen und während der Blutsperredauer zu regulieren. Die verschiedenen Kompressionsmanschetten unterschiedlicher Größe können dabei unterschiedliches Volumen sowie unterschiedliche Formen aufweisen, um eine möglichst gute Anpassung an die Größe und Form der Extremität, dem Patiententypen und dem anzuwendenden Operationsverfahren zu ermöglichen.

Die US2011/0046494 A1 beschreibt eine Blutdruckmanschette, die mit einer elektronischen Komponente ausgestattet ist, welche eine Kodierung von Eigenschaften der Manschette ermöglicht. Die Manschette wird dabei über Verbinder und einen Schlauch mit einem Blutdruckmessgerät verbunden, welches über ein Messgerät verfügt, das die Kodierung der elektronischen Komponente der Manschette erfassen kann. Dadurch kann der Typ und die Größe einer an das Blutdruckmessgerät angeschlossenen Manschette erkannt werden.

Aus der WO03/015,641 A1 ist eine Blutsperreeinrichtung mit einem Tourniquet-Instrument und einer Vielzahl daran anschließbarer Tourniquet-Manschetten bekannt, wobei die Manschetten unterschiedliche Eigenschaften und insbesondere unterschiedliche Manschettengrößen aufweisen und jede Manschette mit einem Manschetten-Verbinder ausgestattet ist, welcher eine Identifizierungseinrichtung umfasst. Die Identifizierungseinrichtung zeigt dabei die physikalischen Eigenschaften der Tourniquet-Manschette an, insbesondere deren Manschettengröße. Das Tourniquet-Instrument umfasst eine Erfassungseinrichtung, die auf die Identifizierungseinrichtung der Manschette anspricht, wenn eine Manschette an dem Tourniquet-Instrument angeschlossen ist, um die physikalischen Eigenschaften der angeschlossenen Manschette zu erfassen. Bei der Identifizierungseinrichtung handelt es sich um eine Auswahl vorbestimmter Farben, wobei jede Farbe einer vorbestimmten physikalischen Eigenschaft der Manschette entspricht, bspw. einer bestimmten Manschettengröße. An dem Tourniquet-Instrument ist eine optische Erfassungseinrichtung angeordnet, welche die Farbe der Identifizierungseinrichtung einer Manschette ermitteln und auf diese Weise auf den Typ und die physikalischen Eigenschaften der angeschlossenen Manschette schließen kann. Die Identifikation der physikalischen Eigenschaften der angeschlossenen Manschette ermöglicht eine Optimierung verschiedener Betriebsparameter sowie die Ausgabe von Warnsignalen und die Speicherung von Betriebsdaten. Darüber hinaus unterbindet die automatische Manschettenidentifikation einen fehlerhaften Anschluss einer ungeeigneten Manschette an das Tourniquet-Instrument.

Diese bekannte Blutsperreeinrichtung mit automatischer Manschettenidentifikation erweist sich jedoch im praktischen Gebrauch als fehleranfällig, weil es durch die optische Erfassungseinrichtung zu einer fehlerhaften Erkennung der Farbe der Identifizierungseinrichtung am Manschetten-Verbinder kommen kann. Es besteht auch die Gefahr, dass eine falsche Identifizierungseinrichtung am Manschetten-Verbinder angebracht wird, was ebenfalls zu einer fehlerhaften Identifizierung der an dem Tourniquet-Instrument angeschlossenen Manschette führt.

Die bekannte Blutsperreeinrichtung ermöglicht über die automatische Manschettenidentifikation zwar die Optimierung verschiedener Betriebsparameter und die Abgabe von Warnsignalen, falls eine ungeeignete Manschette an das Tourniquet-Instrument angeschlossen worden ist. Ein effizienter Betrieb der Blutsperreeinrichtung, der sich bspw. in einem möglichst schnellen Aufblasen und Ablassen der an das Blutsperregerät angeschlossenen Kompressionsmanschette darstellt, ist durch die bekannte Blutsperreeinrichtung jedoch noch nicht ermöglicht.

Hiervon ausgehend liegt der Erfindung die Aufgabe zugrunde, eine Blutsperreeinrichtung aufzuzeigen, die ein möglichst effizientes und insbesondere schnelles Aufblasen der Blutsperremanschette und gleichzeitig einen möglichst fehlerfreien Betrieb der Blutsperreeinrichtung ermöglicht.

Diese Aufgaben werden mit der Steuereinrichtung für ein Blutsperregerät mit den Merkmalen des Anspruchs 1, sowie mit der Verwendung einer Steuereinrichtung zur Steuerung eines Blutsperregeräts mit den Merkmalen des Anspruchs 2 gelöst.

Die Blutsperreeinrichtung gemäß der Erfindung umfasst ein Blutsperregerät mit einer Aufblaseinrichtung und einer Steuereinrichtung sowie eine Kompressionsmanschette, welche an die Aufblaseinrichtung des Blutsperregeräts anschließbar und an ein Körperglied, insbesondere im Bereich der oberen und unteren Extremitäten, anlegbar ist. Die Steuereinrichtung enthält einen Erkennungsmechanismus, mit dem die Größe und insbesondere das Volumen der an die Aufblaseinrichtung angeschlossenen Kompressionsmanschette erfasst werden kann. Die Steuereinrichtung steuert die Aufblaseinrichtung zum Aufblasen der Kompressionsmanschette unter Verwendung eines Parametersatzes, welcher der Größe bzw. dem Volumen der angeschlossenen Kompressionsmanschette zugeordnet ist.

An das Blutsperregerät können unterschiedliche Kompressionsmanschetten mit unterschiedlicher Manschettengröße bzw. unterschiedlichem Volumen angeschlossen werden. Die Kompressionsmanschetten mit unterschiedlicher Manschettengröße bilden dabei einen dem Blutsperregerät zugeordneten Manschettensatz. Jeder Kompressionsmanschette ist dabei ein Parametersatz zur Steuerung eines Aufblasvorgangs dieser Kompressionsmanschette zugeordnet und der Parametersatz ist in einem Datenspeicher der Steuereinrichtung gespeichert. Nach Erfassen der Größe bzw. des Volumens der an das Blutsperregerät angeschlossenen Kompressionsmanschette mittels des Erkennungsmechanismus wählt die Steuereinrichtung den Parametersatz aus, welcher der angeschlossenen Kompressionsmanschette zugeordnet ist und steuert die Aufblaseinrichtung unter Verwendung dieses Parametersatzes, der spezifisch auf die Größe bzw. das Volumen der angeschlossenen Kompressionsmanschette abgestimmt ist. Dadurch kann ein sehr effizientes Aufblasen der angeschlossenen Kompressionsmanschette in möglichst kurzer Zeit erfolgen und es kann verhindert werden, dass die angeschlossene Kompressionsmanschette mit einem falschen Druckprofil oder auf einen nicht geeigneten Enddruck aufgeblasen wird.

Bei dem Erkennungsmechanismus zur Erfassung des Volumens bzw. der Größe der angeschlossenen Kompressionsmanschette wird bevorzugt ein Druck in der Kompressionsmanschette und/oder die Durchflussmenge eines Druckmittels gemessen, welches von der Aufblaseinrichtung in die Kompressionsmanschette gepumpt wird. Bei dem Druckmittel handelt es sich zweckmäßig um Druckluft oder ein anderes komprimiertes Gas, wie z.B. Stickstoff.

In einem bevorzugten Ausführungsbeispiel der Erfindung, in dem die Manschettengröße über eine Druckmessung erfasst wird, steuert die Steuereinrichtung beim Ablauf des Erkennungsmechanismus die Aufblaseinrichtung derart, dass diese die angeschlossene Kompressionsmanschette zunächst über eine vorgegebene Erfassungs-Zeitdauer unter Verwendung eines ersten Parametersatzes aufbläst, der für die kleinste Kompressionsmanschette des Manschettensatzes vorgesehen ist. Nach Ablauf der Erfassungs-Zeitdauer wird der Druck in der angeschlossenen Kompressionsmanschette erfasst und der Steuereinrichtung zugeleitet. Die Steuereinrichtung ermittelt dann aus dem erfassten Druck die Größe bzw. das Volumen der angeschlossenen Kompressionsmanschette und wählt schließlich zum weiteren Aufblasen der Manschette den Parametersatz aus, welcher der Manschette mit dem erfassten Manschettenvolumen zugeordnet ist. Hierfür enthält die Steuereinrichtung einen Datenspeicher, in dem unterschiedliche Parametersätze zum Aufblasen von Kompressionsmanschetten mit unterschiedlicher Größe bzw. unterschiedlichem Volumen gespeichert sind. Die den verschiedenen Kompressionsmanschetten zugeordneten Parametersätze enthalten zweckmäßig ein Aufblasprofil in Form einer Druck-Zeit-Kurve sowie einen Maximaldruck, der beim Aufblasen der Kompressionsmanschette nicht überschritten werden darf. Zweckmäßig enthält der Parametersatz jeder Kompressionsmanschette, welche an das Blutsperregerät anschließbar ist, darüber hinaus auch einen Wert für eine maximale Zeitdauer, welche die Kompressionsmanschette im aufgeblasenen Zustand nicht überschreiten darf. Hierdurch kann gewährleistet werden, dass die durch die aufgeblasene Kompressionsmanschette bewirkte Blutsperre in dem Körperglied, an dem die Kompressionsmanschette angelegt ist, nicht über eine zu lange Zeitdauer aufrecht erhalten wird. Dadurch können Schädigungen im Gewebe des Körperglieds vermieden werden, welche bei einer übermäßig lang andauernden Blutsperre eintreten können.

Diese und weitere Vorteile der Erfindung gehen aus dem nachfolgend unter Bezugnahme auf die begleitenden Zeichnungen näher beschriebenen Ausführungsbeispiel hervor. Die Zeichnungen zeigen:
- **Fig. 1:**: Schematische Darstellung einer erfindungsgemäßen Blutsperreeinrichtung, welche ein Blutsperregerät und eine daran anschließbare Kompressionsmanschette umfasst;
- **Fig. 2:**: Pneumatisches Blockschaltbild eines erfindungsgemäß gesteuerten Blutsperregeräts in einer ersten Ausführungsform;
- **Fig. 3:**: Pneumatisches Blockschaltbild eines erfindungsgemäß gesteuerten Blutsperregeräts in einer zweiten Ausführungsform;
- **Fig. 4:**: Pneumatisches Blockschaltbild eines erfindungsgemäß gesteuerten Blutsperregeräts in einer dritten Ausführungsform;
- **Fig. 5:**: Schematische Darstellung eines Erkennungsmechanismus zur Erfassung der Manschettengröße bzw. des Volumens einer Kompressionsmanschette mittels einer Durchflussmessung.

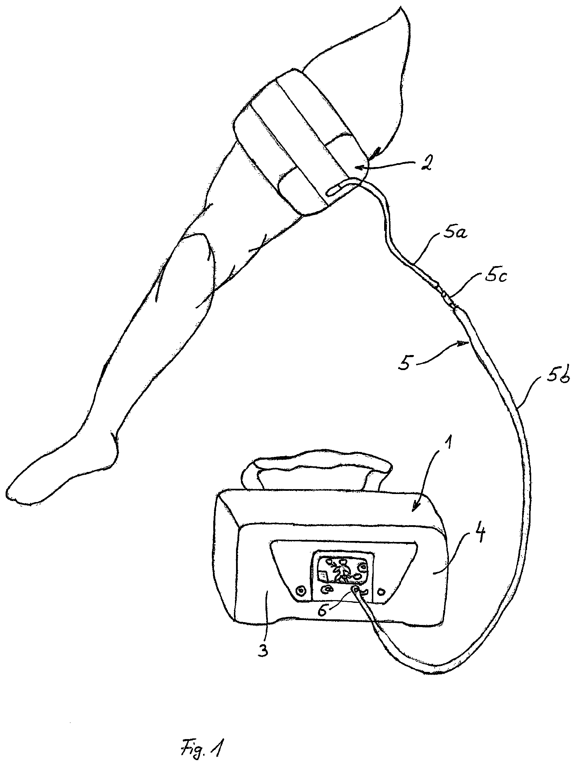

Die schematische Darstellung der Figur 1 zeigt eine Blutsperreeinrichtung mit einem Blutsperregerät 1 und einer daran angeschlossenen Kompressionsmanschette 2, welche an ein Körperglied eines Patienten angelegt ist. In dem zeichnerisch dargestellten Ausführungsbeispiel ist die aufblasbare Kompressionsmanschette 2 an einem Oberschenkel eines Patienten angelegt. In entsprechender Weise kann die Kompressionsmanschette auch an anderen Körperextremitäten (wie z.B. an einem Arm) angelegt werden, um dort eine Regulierung des arteriellen Blutflusses bis hin zu einer absoluten Blutsperre zu bewirken. Das Blutsperregerät 1 enthält eine Aufblaseinrichtung 3 und eine Steuereinrichtung 4, welche in einem Gehäuse angeordnet sind. Die Aufblaseinrichtung 3 ist mit der Steuereinrichtung 4 gekoppelt und dient zum Aufblasen der Kompressionsmanschette 2, welche über einen Verbindungsschlauch 5 mit dem Blutsperregerät 1 verbunden ist. Bei dem Verbindungsschlauch 5 kann es sich um einen Druckluftschlauch, bspw. einem Kunststoffschlauch handeln. Der Verbindungsschlauch 5 kann dabei einteilig oder auch mehrteilig mit einem ersten Schlauchstück 5a, welches an der Kompressionsmanschette 2 angeordnet ist, und einem zweiten Schlauchsstück 5b ausgebildet sein, wobei die beiden Schlauchstücke 5a und 5b über ein Verbindungsstück 5c miteinander verbunden sind. Ein Ende des Verbindungsschlauchs 5 ist mit einem Druckmittelanschluss 6 des Blutsperregeräts 1 verbunden. Das andere Ende des Verbindungsschlauchs 5 ist mit einer aufblasbaren Kammer der Kompressionsmanschette 2 verbunden. Zum Aufblasen der Kompressionsmanschette 2 pumpt die Aufblaseinrichtung 3 des Blutsperregeräts 1 ein Druckmittel, bspw. Druckluft, durch den Verbindungsschlauch 5 in die Kompressionsmanschette 2. Die Aufblaseinrichtung 3 wird dabei von der Steuereinrichtung 4 gesteuert, um die Kompressionsmanschette 2 mit einem geeigneten Aufblasprofil aufzublasen.

An das Blutsperregerät 1 können auf diese Weise verschiedene Kompressionsmanschetten unterschiedlicher Größe angeschlossen werden. So kann insbesondere eine erste Kompressionsmanschette 2 an das Blutsperregerät 1 angeschlossen werden, welche für die Erzeugung einer Blutsperre an einem Körperglied mit großem Umfang, wie z.B. einem Oberschenkel, vorgesehen ist und es kann eine zweite Kompressionsmanschette mit kleinerem Volumen an das Blutsperregerät 1 angeschlossen werden, welche für die Erzeugung einer Blutsperre an einem Körperglied mit kleinerem Umfang, bspw. einem Arm, geeignet ist. Zweckmäßig ist dem Blutsperregerät 1 ein Satz von Kompressionsmanschetten unterschiedlicher Größe und insbesondere mit unterschiedlichem Volumen zugeordnet, um für jeden vorgesehenen Anwendungsfall eine geeignete Kompressionsmanschette mit passender Größe auswählen zu können. Ein solcher Satz von Kompressionsmanschetten kann bspw. drei Manschetten mit unterschiedlicher Größe bzw. unterschiedlichem Volumen umfassen. Die verschiedenen Manschettengrößen können dabei in bekannter Weise ihrer Größe entsprechend gekennzeichnet sein, bspw. mit einem Buchstabencode "S" für eine kleine Manschette, "M" für eine mittlere Manschette und "L" für eine große Manschette. Jeder Kompressionsmanschette (2S, 2M, 2L) des Manschettensatzes ist dabei ein spezifisches Aufblasprofil zugeordnet. Das Aufblasprofil jeder Manschette ist in einem manschettenspezifischen Parametersatz enthalten. Die Parametersätze für die einzelnen Kompressionsmanschetten unterschiedlicher Größe sind in einem Datenspeicher der Steuereinrichtung 4 des Blutsperregeräts 1 hinterlegt. Zweckmäßig enthält jeder Parametersatz, der einer Manschette mit einer bestimmten Größe bzw. einem bestimmten Volumen zugeordnet ist, ein Aufblasprofil in Form einer Druck-Zeit-Kurve und/oder einer zeitabhängigen Durchflussmenge des zum Aufblasen der Manschette verwendeten Druckmittels. Darüber hinaus kann der jeder Kompressionsmanschette zugeordnete Parametersatz einen Wert für einen Maximaldruck enthalten, der beim Aufblasen der jeweiligen Kompressionsmanschette nicht überschritten werden darf. Ferner kann der Parametersatz jeder Kompressionsmanschette einen Wert für eine maximale Blutsperre-Zeitdauer enthalten, den die Kompressionsmanschette im aufgeblasenen Zustand nicht überschreiten darf. Jeder Manschette mit einem bestimmten Volumen ist eindeutig ein entsprechender Parametersatz zugeordnet.

Die in dem Blutsperregerät 1 integrierte Steuereinrichtung 4 enthält einen Erkennungsmechanismus, mit dem die Größe bzw. das Volumen der an das Blutsperregerät 1 angeschlossenen Kompressionsmanschette 2 erfasst werden kann. In einem bevorzugten Ausführungsbeispiel ermittelt der Erkennungsmechanismus die Größe bzw. das Volumen der angeschlossenen Kompressionsmanschette 2 über eine Druckmessung. In einem alternativen Ausführungsbeispiel kann die Erfassung der Größe bzw. des Volumens der angeschlossenen Kompressionsmanschette 2 auch mittels einer Durchflussmessung erfolgen. Die Einzelheiten dieser beiden Ausführungsvarianten des Erkennungsmechanismus werden nachfolgend noch im Einzelnen beschrieben.

Die von dem Erkennungsmechanismus erfasste Größe der angeschlossenen Kompressionsmanschette 2 bzw. das vom Erkennungsmechanismus erfasste Volumen der angeschlossenen Kompressionsmanschette 2 wird der Steuereinrichtung 4 zugeleitet. Die Steuereinrichtung 4 wählt anhand der erfassten Größe bzw. des erfassten Volumens der angeschlossenen Kompressionsmanschette 2 den passenden Parametersatz für diese Manschette aus dem Datenspeicher, in dem die Parametersätze für alle anschließbaren Manschetten hinterlegt sind, aus und steuert die Aufblaseinrichtung 3 unter Verwendung des ausgewählten Parametersatzes. Die Aufblaseinrichtung 3 pumpt entsprechend dem ausgewählten Parametersatz und insbesondere gemäß dem darin enthaltenen Aufblasprofil die angeschlossene Kompressionsmanschette auf, so dass diese in dem Körperglied, an welches sie angelegt ist, den arteriellen Blutfluss regelt.

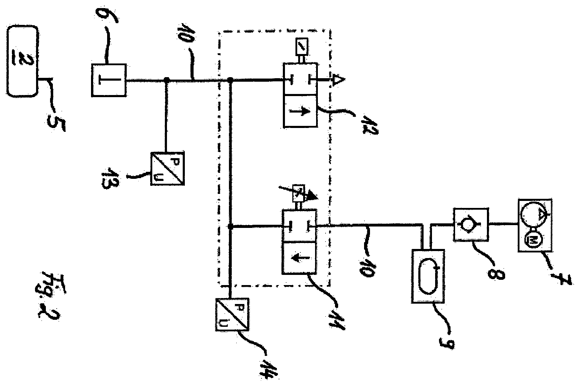

In den Figuren 2 - 4 sind verschiedene Ausführungsformen des Blutsperregeräts 1 als Pneumatik-Blockschaltbilder dargestellt. Die in Figur 2 gezeigte Ausführungsform zeigt ein Blutsperregerät 1 mit einer einkanaligen Aufblaseinrichtung 3, welche einen Kompressor 7 umfasst. Der Kompressor 7, der entweder über einen Stromanschluss oder einen im Blutsperregerät 1 integrierten Akkumulator mit Energie versorgt wird, pumpt ein Druckmittel, insbesondere Druckluft, über ein Rückschlagventil 8 in einen Druckmittelspeicher 9 und von dort über eine Druckmittelleitung 10 zu einem Anschluss 6, an den der Verbindungsschlauch 5 einer Kompressionsmanschette 2 anschließbar ist. In der Druckmittelleitung 10 ist ein Proportionalventil 11 und ein Entlüftungsventil 12 angeordnet. Das Proportionalventil 11 dient zur Regelung des am Anschluss 6 anstehenden Drucks. Über das Entlüftungsventil 12 kann der in der Kompressionsmanschette 2 aufgebaute Druck abgelassen werden, um die von der aufgeblasenen Kompressionsmanschette 2 bewirkte Blutsperre wieder aufzuheben. Der vom Kompressor 7 am Anschluss 6 bereitgestellte Druck wird mittels eines Druckmessgeräts 13 erfasst. Bei an den Anschluss 6 angeschlossener Kompressionsmanschette 2 entspricht dieser Druck dem Manschettendruck. Der nach dem Proportionalventil 11 anliegende Proportionaldruck wird mit einem weiteren Druckmessgerät 14 erfasst.

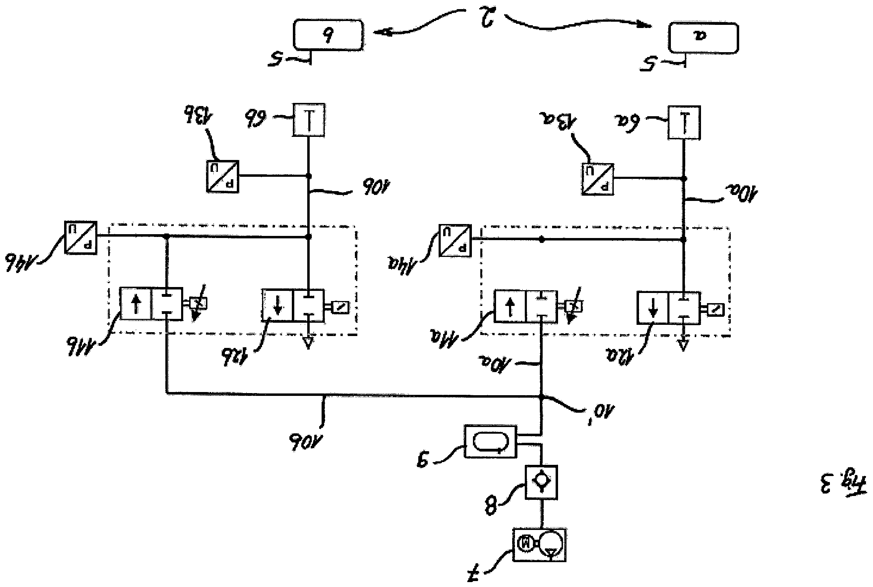

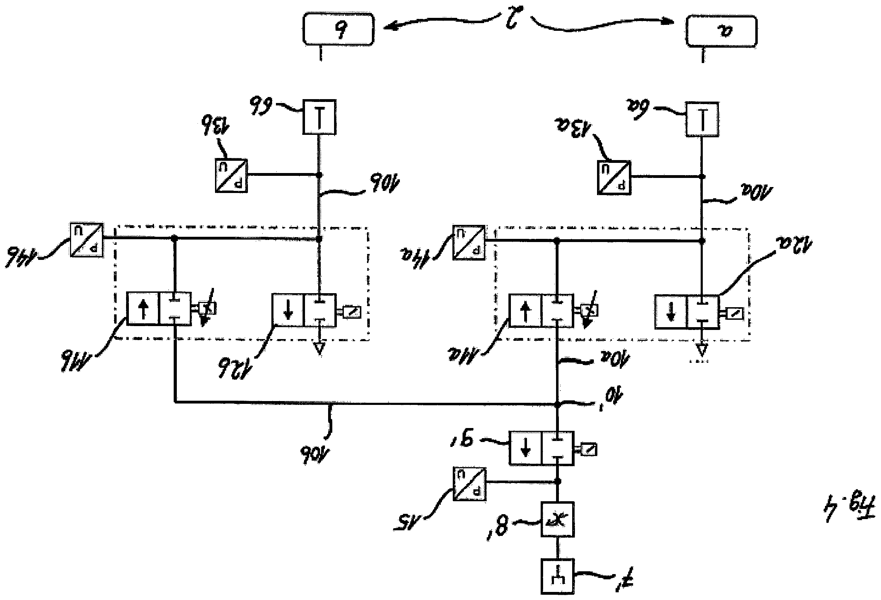

In den Figuren 3 und 4 sind zwei Ausführungsbeispiele eines Blutsperregeräts 1 mit einer zweikanaligen Aufblaseinrichtung 3 in einem Pneumatik-Blockschaltbild dargestellt. Solche Blutsperregeräte mit einer zweikanaligen Aufblaseinrichtung können zum Aufblasen von Zwei-Kammer-Manschetten verwendet werden. Das zweikanalige Blutsperregerät 1 enthält dabei zwei Anschlüsse 6a, 6b zum Anschluss der beiden Kammern a und b einer Zwei-Kammer-Manschette 2. Jedem Anschluss 6a, 6b ist ein Proportionalventil 11a, 11b zur Regelung des am jeweiligen Anschluss anstehenden Drucks sowie ein Entlüftungsventil 12a, 12b zugeordnet. Entsprechend verfügt jeder Anschluss 6a, 6b über ein Druckmessgerät 13a, 13b zur Erfassung des Kammerdrucks der am jeweiligen Anschluss angeschlossenen Kammer a, b der Manschette 2. Jedem Proportionalventil 11a, 11b ist ferner ein weiteres Druckmessgerät 14a, 14b zur Erfassung des Proportionaldrucks zugeordnet. Wie in dem Ausführungsbeispiel von Figur 2 verfügt auch das Blutsperregerät 1 des Ausführungsbeispiels von Figur 3 über einen Druckmittelspeicher 9 und einen Kompressor 7 sowie ein dazwischen angeordnetes Rückschlagventil 8 sowie eine Druckmittelleitung 10, über welche das Druckmittel vom Druckmittelspeicher 9 zu den Anschlüssen 6a und 6b geführt wird. Zur Aufteilung des im Druckmittelspeicher 9 bevorrateten Druckmittels an die beiden Anschlüsse 6a und 6b ist in der Druckmittelleitung 10 in dem Ausführungsbeispiel der Figur 3 eine Verzweigung 10' in zwei Druckmittelleitungen 10a und 10b vorgesehen.

Das in Figur 4 gezeigte Ausführungsbeispiel des Blutsperregeräts 1 entspricht im Wesentlichen dem Ausführungsbeispiel von Figur 3. Anders als bei dem Ausführungsbeispiel der Figur 3 umfasst das Blutsperregerät 1 bei dem Ausführungsbeispiel von Figur 4 jedoch keinen Kompressor, sondern vielmehr einen Anschluss 7' an eine zentrale Gasversorgung, welche ein komprimiertes Gas als Druckmittel bereitstellt. An den Druckmittelanschluss 7' schließt sich ein Druckminderer 8' und ein Ventil 9' an. Der nach dem Druckminderer 8' anstehende Arbeitsdruck wird von einem weiteren Druckmessgerät 15 erfasst.

Die in den Figuren 2 - 4 gezeigten Ausführungsbeispiele des Blutsperregeräts 1 weisen einen Erkennungsmechanismus auf, über den mittels einer Druckmessung die Größe bzw. das Volumen der angeschlossenen Kompressionsmanschette 2 erfasst werden kann. Dazu wird beim Ablauf des Erkennungsmechanismus die am Anschluss 6 angeschlossene Kompressionsmanschette 2 zunächst über eine vorgegebene Erfassungs-Zeitdauer t1 unter Verwendung eines ersten Parametersatzes aufgeblasen. Der erste Parametersatz entspricht dabei dem Parametersatz, welcher der kleinsten anschließbaren Kompressionsmanschette 2S des Manschettensatzes zugeordnet ist. Nach Ablauf der Erfassungs-Zeitdauer t1 wird der in der angeschlossenen Kompressionsmanschette 2 erzeugte Druck mittels des Druckmessgeräts 13 erfasst. Wenn eine Zwei-Kammer-Manschette oder zwei einzelne Manschetten an das Blutsperregerät 1 angeschlossen ist, kann der sich beim Aufblasen der beiden Kammern a, b ausgebildete Kammerdruck in jeder Kammer a, b getrennt mittels der Druckmessgeräte 13a und 13b erfasst werden. Aus dem erfassten Manschettendruck, der sich nach Ablauf der Erfassungs-Zeitdauer t1 in der Kompressionsmanschette 2 ausgebildet hat, kann auf die Größe und insbesondere das Volumen der angeschlossenen Manschette geschlossen werden.

Liegt der erfasste Manschettendruck in der angeschlossenen Kompressionsmanschette 2 über einem vorgegebenen ersten Minimaldruck p1, so handelt es sich bei der angeschlossenen Kompressionsmanschette 2 um die kleinste anschließbare Manschette des dem Blutsperregerät 1 zugeordneten Manschettensatzes. Die Steuereinrichtung 4 des Blutsperregeräts 1 wählt in diesem Fall für das weitere Aufblasen der angeschlossenen Manschette den Parametersatz aus, welcher der Manschette mit der kleinsten Größe bzw. dem kleinsten Volumen zugeordnet ist und bläst die angeschlossene Kompressionsmanschette 2 unter Verwendung dieses Parametersatzes über eine Aufblasdauer t2 weiter auf, bis der gemäß diesem Parametersatz vorgesehene Enddruck erreicht ist.

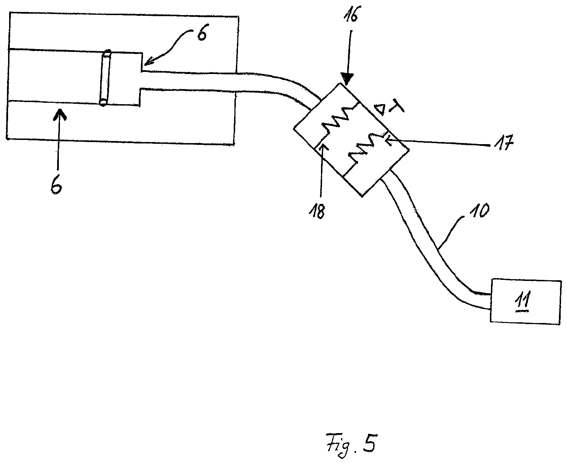

Liegt der beim Ablauf des Erkennungsmechanismus erfasste Manschettendruck dagegen unter dem vorgegebenen ersten Minimaldruck p1, so kann daraus geschlossen werden, dass nicht die kleinste sondern eine größere Manschette (2M oder 2L) an dem Blutsperregerät angeschlossen ist. In diesem Fall bläst die Aufblaseinrichtung 3 die angeschlossene Kompressionsmanschette 2 über eine vorgegebene erste Aufblas-Zeitdauer t2 unter Verwendung eines zweiten Parametersatzes auf, der für die zweitkleinste Kompressionsmanschette (2M) des Manschettensatzes vorgesehen ist. Nach Ablauf der ersten Aufblas-Zeitdauer t2 wird wiederum der sich in der angeschlossenen Kompressionsmanschette 2 ausgebildete Manschettendruck mittels des Druckmessgeräts 13 erfasst. Liegt der erfasste Manschettendruck oberhalb eines zweiten Minimaldrucks p2, der größer ist als der erste Minimaldruck p1, so handelt es sich bei der angeschlossenen Kompressionsmanschette 2 um die zweitkleinste Manschette des Manschettensatzes. Für das weitere Aufblasen der angeschlossenen Kompressionsmanschette 3 wählt die Steuereinrichtung in diesem Fall dann den Parametersatz aus, welcher dieser Manschette mit dem zweitkleinsten Volumen zugeordnet ist und bläst die angeschlossene Manschette unter Verwendung dieses Parametersatzes bis zum vorgegebenen Enddruck auf. Liegt der erfasste Manschettendruck dagegen unterhalb des zweiten Minimaldrucks p2, lässt sich darauf schließen, dass die nächstgrößere Kompressionsmanschette an dem Blutsperregerät 1 angeschlossen ist. Umfasst der dem Blutsperregerät 1 zugeordnete Manschettensatz insgesamt drei Manschetten (2S, 2M, 2L) unterschiedlicher Größe (S, M, L), ist in diesem Fall also die größte Manschette (2L) dieses Manschettensatzes angeschlossen. Die Steuereinrichtung wählt in diesem Fall dann für das weitere Aufblasen der angeschlossenen Manschette den der größten Manschette zugeordneten Parametersatz aus und bläst die angeschlossene Manschette entsprechend dem Aufblasprofil dieses Parametersatzes bis zum vorgesehenen Enddruck auf. In Figur 5 ist eine weitere Ausführungsform eines Blutsperregeräts mit einem Erkennungsmechanismus zur Erkennung der angeschlossenen Kompressionsmanschette gezeigt. Bei diesem Ausführungsbeispiel erfolgt die Erkennung der Größe bzw. des Volumens der an das Blutsperregerät angeschlossenen Kompressionsmanschette über eine Durchflussmessung. Hierfür ist in der Druckmittelleitung 10 zwischen dem Proportionalventil 11 und dem Anschluss 6 ein Durchflussmesser 16 angeordnet. Der Durchflussmesser 16 erfasst die durch die Druckmittelleitung 10 zum Anschluss 6 strömende Menge des Druckmittels. Aus der pro Zeiteinheit durchströmenden Menge kann auf das Volumen der am Anschluss 6 über einen nicht dargestellten Verbindungsschlauch angeschlossene Kompressionsmanschette (welche in Figur 6 ebenfalls nicht gezeigt ist) geschlossen werden.

Der Durchflussmesser 16 kann bspw. als kalorimetrischer bzw. thermischer Durchflussmesser ausgebildet sein. Der in Figur 6 gezeigte Durchflussmesser 16 umfasst einen ersten Widerstandsdraht 17 und einen zweiten Widerstandsdraht 18, welche jeweils als Temperaturfühler wirken. Der erste Widerstandsdraht 17 wird dabei zunächst mit einem konstanten elektrischen Strom beaufschlagt, wodurch er sich erwärmt und gegenüber dem anderen Widerstandsdraht 18, der die aktuelle Temperatur in der Druckmittelleitung 10 erfasst, eine vorgegebene Temperaturdifferenz ΔT erzeugt wird, wenn kein Druckmittel durch die Druckmittelleitung 10 strömt. Strömt das Druckmittel (komprimiertes Gas) dagegen durch die Druckmittelleitung 10 wird dem beheizten Widerstandsdraht 17 durch das vorbeiströmende Gas Wärme entzogen und durch die Strömung abgeführt. Die dadurch erfolgende Abkühlung wird erfasst und durch Nachregelung des durch den ersten Widerstandsdraht 17 geleiteten Heizstroms ausgeglichen, bis die voreingestellte Temperaturdifferenz ΔT wieder eingestellt ist. Die erfolgte Nachregelung des Heizstroms stellt damit ein Maß für die Abkühlung des ersten Widerstandsdrahts 17 durch das vorbeiströmende Gas dar, welche wiederum direkt proportional zur Menge des vorbeigeströmten Gases ist. Aus dem Wert der Nachregelung des Heizstroms kann damit auf den pro Zeiteinheit in der Druckmittelleitung durchgeströmten Massestrom des Gases geschlossen werden.

Der Durchflussmesser 16 erfasst auf diese Weise den Massestrom des durch die Druckmittelleitung 10 zum Anschluss 6 strömenden Druckmittels. Da dieser vom Volumen der am Anschluss 6 angeschlossenen Kompressionsmanschette abhängig ist, kann durch die Erfassung des pro Zeiteinheit in die angeschlossene Kompressionsmanschette strömenden Massestroms auf das Volumen der Kompressionsmanschette geschlossen werden.

Das erfindungsgemäße Steuersystem mit dem Erkennungsmechanismus zur Erfassung der Größe bzw. des Volumens der an das Blutsperregerät 1 angeschlossenen Kompressionsmanschette 2 gewährleistet, dass die jeweils angeschlossene Kompressionsmanschette immer mit dem ihr zugeordneten Aufblasprofil aufgeblasen wird. Dies verhindert zum einen Fehlfunktionen und insbesondere ein zu starkes Aufblasen einer kleinen Kompressionsmanschette. Ferner ermöglicht das erfindungsgemäße Steuersystem ein schnelleres Aufblasen der angeschlossenen Kompressionsmanschette, wenn beim Aufblasen deren Größe berücksichtigt wird und ein der Manschettengröße angepasstes Aufblasprofil verwendet werden kann. Schließlich können auch Schädigungen des Gewebes in dem Körperglied des Patienten vermieden werden, wenn die angelegte Kompressionsmanschette mit einem der Manschettengröße angepassten und optimierten Aufblasprofil aufgeblasen wird.

Die Erfindung ist nicht auf das beschriebene Ausführungsbeispiel beschränkt. So kann bspw. der Manschettensatz mehr als drei Kompressionsmanschetten mit unterschiedlicher Größe umfassen. Der Erkennungsmechanismus zur Erfassung der Manschettengröße über eine Druckmessung wird in diesem Fall dann iterativ wiederholt, wenn eine der größeren Manschetten des Manschettensatzes angeschlossen sein sollte. Bei den Kompressionsmanschetten kann es sich um "Dual-Port"-Manschetten handeln, welche neben einem ersten Druckmittelanschluss zum Aufblasen der Manschette einen zweiten Anschluss zur Erfassung des Manschettendrucks aufweisen.

## Patentansprüche

1. Steuereinrichtung (4) für ein Blutsperregerät (1), an welches eine erste Kompressionsmanschette (2) und/oder wenigstens eine zweite Kompressionsmanschette anschließbar ist, wobei die anschließbaren Kompressionsmanschetten (2) unterschiedliches Volumen aufweisen und das Blutsperregerät (1) eine Aufblaseinrichtung (3) zum Aufblasen der angeschlossenen Kompressionsmanschette (2) umfasst, **dadurch gekennzeichnet, dass** jeder Kompressionsmanschette (2) ein Parametersatz zur Steuerung eines Aufblasvorgangs der jeweiligen Kompressionsmanschette (2) zugeordnet und in einem Datenspeicher der Steuereinrichtung (4) gespeichert ist, wobei der jeder Kompressionsmanschette (2) zugeordnete Parametersatz abhängig vom Volumen der Kompressionsmanschette (2) ist und ein zeitabhängiges Aufblasprofil für das Aufblasen der Kompressionsmanschette definiert.

2. Steuereinrichtung nach Anspruch 1, **gekennzeichnet durch** einen Erkennungsmechanismus, mit dem das Volumen der an der Aufblaseinrichtung (3) angeschlossenen Kompressionsmanschette (2) erfasst wird.

3. Steuereinrichtung nach Anspruch 2, **dadurch gekennzeichnet, dass** bei dem Erkennungsmechanismus zur Erfassung des Volumens der angeschlossenen Kompressionsmanschette (2) ein Druck in der Kompressionsmanschette (2) und/oder die Durchflussmenge eines Druckmittels in die Kompressionsmanschette (2) gemessen wird.

4. Steuereinrichtung nach Anspruch 2, **wobei** die Steuereinrichtung (4) beim Ablauf des Erkennungsmechanismus die Aufblaseinrichtung (3) so steuert, dass diese die an der Aufblaseinrichtung (3) angeschlossene Kompressionsmanschette (2) zunächst über eine vorgegebene Erfassungs-Zeitdauer (t1) unter Verwendung eines ersten Parametersatzes aufbläst, der für die kleinste anschließbare Kompressionsmanschette vorgesehen ist, nach Ablauf dieser Erfassungs-Zeitdauer (t1) der Druck in der angeschlossenen Kompressionsmanschette erfasst und der Steuereinrichtung (4) zugeleitet wird und die Steuereinrichtung (4) aus dem erfassten Druck das Volumen der angeschlossenen Kompressionsmanschette ermittelt.

5. Steuereinrichtung nach Anspruch 3, **wobei** die Steuereinrichtung (4) nach der Ermittlung des Volumens der angeschlossenen Kompressionsmanschette (2) die Aufblaseinrichtung (3) so steuert, dass diese die angeschlossene Kompressionsmanschette über eine vorgegebene Aufblas-Zeitdauer (t2) unter Verwendung des ersten Parametersatzes aufbläst, wenn beim Ablauf des Erkennungsmechanismus der erfasste Druck in der angeschlossenen Kompressionsmanschette einen vorgegebenen ersten Minimaldruck (p1) überschritten hat.

6. Steuereinrichtung nach Anspruch 3 oder 4, **wobei** die Steuereinrichtung (4) nach der Erfassung des Volumens der angeschlossenen Kompressionsmanschette (2) die Aufblaseinrichtung (3) so steuert, dass diese die angeschlossene Kompressionsmanschette über eine vorgegebene erste Aufblas-Zeitdauer (t2) unter Verwendung eines zweiten Parametersatzes aufbläst, der für die zweitkleinste anschließbare Kompressionsmanschette vorgesehen ist, wenn beim Ablauf des Erkennungsmechanismus der erfasste Druck in der angeschlossenen Kompressionsmanschette den vorgegebenen ersten Minimaldruck (p1) unterschritten hat.

7. Steuereinrichtung nach Anspruch 5, **wobei** nach Ablauf der ersten Aufblas-Zeitdauer (t2) der Druck in der angeschlossenen Kompressionsmanschette (2) erfasst und der Steuereinrichtung (4) zugeleitet wird und die Steuereinrichtung (4) aus diesem Druck das Volumen der angeschlossenen Kompressionsmanschette ermittelt und die Aufblaseinrichtung so steuert, dass diese die angeschlossene Kompressionsmanschette über eine vorgegebene zweite Aufblas-Zeitdauer (t3) unter Verwendung eines dritten Parametersatzes aufbläst, der für die dritttkleinste anschließbare Kompressionsmanschette vorgesehen ist, wenn nach Ablauf der ersten Aufblas-Zeitdauer (t2) der erfasste Druck in der angeschlossenen Kompressionsmanschette einen vorgegebenen zweiten Minimaldruck (p2) unterschritten hat.

8. Steuereinrichtung nach Anspruch 4 oder 5, **dadurch gekennzeichnet, dass** die Verfahrensschritte der Ansprüche 5 und 6 iterativ fortgesetzt werden, bis die angeschlossene Kompressionsmanschette (2) auf einen Enddruck aufgeblasen ist, welcher durch den Parametersatz der angeschlossenen Kompressionsmanschette vorgegeben ist.

9. Steuereinrichtung nach einem der Ansprüche 1 bis 8, **dadurch gekennzeichnet, dass** der jeder Kompressionsmanschette zugeordnete Parametersatz einen zeitabhängigen Druckverlauf eines Aufblas- oder Ablassvorgangs zum Aufblasen bzw. Ablassen der Kompressionsmanschette enthält.

10. Steuereinrichtung nach einem der Ansprüche 1 bis 9, **dadurch gekennzeichnet, dass** der jeder Kompressionsmanschette zugeordnete Parametersatz einen Wert für einen Maximaldruck enthält, der beim Aufblasen der Kompressionsmanschette nicht überschritten werden darf.

11. Steuereinrichtung nach einem der Ansprüche 1 bis 10, **dadurch gekennzeichnet, dass** der jeder Kompressionsmanschette zugeordnete Parametersatz einen Wert für eine maximale Zeitdauer enthält, welche die Kompressionsmanschette im aufgeblasenen Zustand nicht überschreiten darf.

12. Blutsperreinrichtung mit einem eine Aufblaseinrichtung (3) und eine Steuereinrichtung (4) nach Anspruch 1 aufweisendes Blutsperregerät (1) und mit einer an ein Körperglied anlegbaren und an die Aufblaseinrichtung anschließbaren Kompressionsmanschette (2), **dadurch gekennzeichnet, dass** ein Erkennungsmechanismus vorgesehen ist, mit dem die Steuereinrichtung (4) die Größe und/oder das Volumen der Kompressionsmanschette (2) erfasst, wenn die Kompressionsmanschette (2) an die Aufblaseinrichtung (3) angeschlossen ist, wobei die Steuereinrichtung (4) die Aufblaseinrichtung (3) zum Aufblasen der Kompressionsmanschette (2) unter Verwendung des Parametersatzes steuert, welcher der vom Erkennungsmechanismus ermittelten Größe und/oder Volumen der angeschlossenen Kompressionsmanschette (2) zugeordnet ist.

13. Blutsperreinrichtung nach Anspruch 12, **dadurch gekennzeichnet, dass** der Erkennungsmechanismus die Größe und/oder das Volumen der angeschlossenen Kompressionsmanschette (2) mittels einer Druckmessung oder mittels einer Durchflussmessung ermittelt, wobei
- bei der Druckmessung der Druck in der der angeschlossenen Kompressionsmanschette (2) erfasst wird, der beim Aufblasen der Kompressionsmanschette mittels der Aufblaseinrichtung (3) über eine vorgegebene Erfassungs-Zeitdauer (t1) erzeugt wird, oder
- bei der Durchflussmessung die Durchflussmenge eines Druckmittels erfasst wird, die beim Aufblasen der Kompressionsmanschette (2) mittels der Aufblaseinrichtung (3) über eine vorgegebene Erfassungs-Zeitdauer (t1) in die angeschlossene Kompressionsmanschette (2) gepumpt wird.

## Claims

1. Control device (4) for a tourniquet (1), to which a first compression cuff (2) and/or at least one second compression cuff is connectable, wherein the compression cuffs (2) which are connectable have different volume and the tourniquet (1) comprises an inflating device (3) for inflating the connected compression cuff (2), **characterised in that** a set of parameters for controlling an inflating process of the respective compression cuff (2) is assigned to each compression cuff (2) and stored in a data memory of the control device (4), wherein the set of parameters assigned to each compression cuff (2) is dependent on the volume of the compression cuff (2) and defines a time-dependent inflating profile for inflating the compression cuff.

2. Control device according to claim 1, **characterised by** a detection mechanism, with which the volume of the compression cuff (2) connected to the inflating device (3) is recorded.

3. Control device according to claim 2, **characterised in that** in the detection mechanism for recording the volume of the connected compression cuff (2), a pressure in the compression cuff (2) and/or the flow rate of a pressure medium into the compression cuff (2) is measured.

4. Control device according to claim 2, wherein during operation of the detection mechanism, the control device (4) controls the inflating device (3) so that it inflates the compression cuff (2) connected to the inflating device (3) first of all over a predetermined recording time period (t1) using a first set of parameters which is provided for the smallest compression cuff which is connectable, after passing of this recording time period (t1), the pressure in the connected compression cuff is recorded and sent to the control device (4) and the control device (4) determines the volume of the connected compression cuff from the pressure recorded.

5. Control device according to claim 3, wherein after determining the volume of the connected compression cuff (2), the control device (4) controls the inflating device (3) so that it inflates the connected compression cuff over a predetermined inflating time period (t2) using the first set of parameters if, during operation of the detection mechanism, the recorded pressure in the connected compression cuff has exceeded a predetermined first minimum pressure (p1).

6. Control device according to claim 3 or 4, wherein after recording the volume of the connected compression cuff (2), the control device (4) controls the inflating device (3) so that it inflates the connected compression cuff over a predetermined first inflating time period (t2) using a second set of parameters which is provided for the second smallest compression cuff which is connectable if, during operation of the detection mechanism, the recorded pressure in the connected compression cuff has fallen short of the predetermined first minimum pressure (p1).

7. Control device according to claim 5, wherein after passing of the first inflating time period (t2), the pressure in the connected compression cuff (2) is recorded and sent to the control device (4) and the control device (4) determines the volume of the connected compression cuff from this pressure and controls the inflating device so that it inflates the connected compression cuff over a predetermined second inflating time period (t3) using a third set of parameters which is provided for the third smallest compression cuff which is connectable if, after passing of the first inflating time period (t2), the recorded pressure in the connected compression cuff has fallen short of a predetermined second minimum pressure (p2).

8. Control device according to claim 4 or 5, **characterised in that** the method steps of claims 5 and 6 are continued iteratively until the connected compression cuff (2) is inflated to a final pressure which is predetermined by the set of parameters of the connected compression cuff.

9. Control device according to one of claims 1 to 8, **characterised in that** the set of parameters assigned to each compression cuff has a time-dependent pressure course of an inflating process or deflating process for inflating or deflating the compression cuff.

10. Control device according to one of claims 1 to 9, **characterised in that** the set of parameters assigned to each compression cuff has a value for a maximum pressure which may not be exceeded when inflating the compression cuff.

11. Control device according to one of claims 1 to 10, **characterised in that** the set of parameters assigned to each compression cuff has a value for a maximum time period which the compression cuff may not exceed in the inflated state.

12. Tourniquet device with a tourniquet (1) having an inflating device (3) and a control device (4) according to claim 1 and with a compression cuff (2) which is placeable on a limb and connectable to the inflating device, **characterised in that** a detection mechanism is provided, with which the control device (4) records the size and/or the volume of the compression cuff (2) if the compression cuff (2) is connected to the inflating device (3), wherein the control device (4) controls the inflating device (3) for inflating the compression cuff (2) using the set of parameters which is assigned to the size and/or volume of the connected compression cuff (2) determined by the detection mechanism.

13. Tourniquet device according to claim 12, **characterised in that** the detection mechanism determines the size and/or the volume of the connected compression cuff (2) by means of a pressure measurement or by means of a flow measurement, wherein
- during the pressure measurement, the pressure in the connected compression cuff (2) is recorded which is generated during inflation of the compression cuff by means of the inflating device (3) over a predetermined recording time period (t1), or
- during the flow measurement, the flow rate of a pressure means is recorded which is pumped into the connected compression cuff (2) during inflation of the compression cuff (2) by means of the inflating device (3) over a predetermined recording time period (t1).

## Revendications

1. Dispositif de commande (4) pour un appareil de réalisation de garrot (1), auquel un premier manchon de compression (2) et/ou au moins un deuxième manchon de compression peut être raccordé, dans lequel les manchons de compression (2) pouvant être raccordés présentent un volume différent et l'appareil de réalisation de garrot (1) comprend un dispositif de gonflage (3) pour le gonflage du manchon de compression (2) raccordé, **caractérisé en ce qu'**un ensemble de paramètres pour la commande d'une opération de gonflage du manchon de compression (2) concerné est associé à chaque manchon de compression (2) et est mis en mémoire dans une mémoire de données du dispositif de commande (4), dans lequel l'ensemble de paramètres associé à chaque manchon de compression (2) est dépendant du volume du manchon de compression (2) et définit un profil de gonflage en fonction du temps pour le gonflage du manchon de compression.

2. Dispositif de commande selon la revendication 1, **caractérisé par** un mécanisme de détection, avec lequel le volume du manchon de compression (2) raccordé au dispositif de gonflage (3) est détecté.

3. Dispositif de commande selon la revendication 2, **caractérisé en ce que** dans le mécanisme de détection pour la détection du volume du manchon de compression (2) raccordé, une pression dans le manchon de compression (2) et/ou le débit d'un moyen de pression dans le manchon de compression (2) est mesuré.

4. Dispositif de commande selon la revendication 2, dans lequel le dispositif de commande (4) lors du déroulement du mécanisme de détection commande le dispositif de gonflage (3) de sorte que celui-ci gonfle le manchon de compression (2) raccordé au dispositif de gonflage (3) d'abord pendant une durée de détection (t1) prédéfinie en utilisant un premier ensemble de paramètres, qui est prévu pour le plus petit manchon de compression pouvant être raccordé, après l'écoulement de cette durée de détection (t1) la pression dans le manchon de compression raccordé est détectée et transmise au dispositif de commande (4) et le dispositif de commande (4) détermine le volume du manchon de compression raccordé à partir de la pression détectée.

5. Dispositif de commande selon la revendication 3, dans lequel le dispositif de commande (4) après la détermination du volume du manchon de compression (2) raccordé commande le dispositif de gonflage (3) de sorte que celui-ci gonfle le manchon de compression raccordé pendant une durée de gonflage (t2) prédéfinie en utilisant le premier ensemble de paramètres, lorsque lors du déroulement du mécanisme de détection la pression détectée dans le manchon de compression raccordé a dépassé une première pression minimum prédéfinie (p1).

6. Dispositif de commande selon la revendication 3 ou 4, dans lequel le dispositif de commande (4) après la détection du volume du manchon de compression (2) raccordé commande le dispositif de gonflage (3) de sorte que celui-ci gonfle le manchon de compression raccordé pendant une première durée de gonflage (t2) prédéfinie en utilisant un deuxième ensemble de paramètres, qui est prévu pour le deuxième plus petit manchon de compression pouvant être raccordé, lorsque lors du déroulement du mécanisme de détection la pression détectée dans le manchon de compression raccordé est passée sous la première pression minimum prédéfinie (p1).

7. Dispositif de commande selon la revendication 5, dans lequel après l'écoulement de la première durée de gonflage (t2) la pression dans le manchon de compression (2) raccordé est détectée et transmise au dispositif de commande (4) et le dispositif de commande (4) détermine le volume du manchon de compression raccordé à partir de cette pression et commande le dispositif de gonflage de sorte que celui-ci gonfle le manchon de compression raccordé pendant une deuxième durée de gonflage (t3) prédéfinie en utilisant un troisième ensemble de paramètres, qui est prévu pour le troisième plus petit manchon de compression pouvant être raccordé lorsqu'après l'écoulement de la première durée de gonflage (t2) la pression détectée dans le manchon de compression raccordé est passée sous une deuxième pression minimum prédéfinie (p2).

8. Dispositif de commande selon la revendication 4 ou 5, **caractérisé en ce que** les étapes de procédé des revendications 5 et 6 sont poursuivies de manière itérative jusqu'à ce que le manchon de compression (2) raccordé soit gonflé à une pression finale, laquelle est prédéfinie par l'ensemble de paramètres du manchon de compression raccordé.

9. Dispositif de commande selon l'une quelconque des revendications 1 à 8, **caractérisé en ce que** l'ensemble de paramètres associé à chaque manchon de compression contient une évolution de la pression en fonction du temps d'une opération de gonflage ou de dégonflage pour le gonflage ou le dégonflage du manchon de compression.

10. Dispositif de commande selon l'une quelconque des revendications 1 à 9, **caractérisé en ce que** l'ensemble de paramètres associé à chaque manchon de compression contient une valeur pour une pression maximum, qui ne doit pas être dépassée lors du gonflage du manchon de compression.

11. Dispositif de commande selon l'une quelconque des revendications 1 à 10, **caractérisé en ce que** l'ensemble de paramètres associé à chaque manchon de compression contient une valeur pour une durée maximum, que le manchon de compression ne doit pas dépasser à l'état gonflé.

12. Dispositif de réalisation de garrot avec un appareil de réalisation de garrot (1) présentant un dispositif de gonflage (3) et un dispositif de commande (4) selon la revendication 1 et avec un manchon de compression (2) applicable sur un membre du corps et pouvant être raccordé au dispositif de gonflage, **caractérisé en ce qu'**un mécanisme de détection est prévu, avec lequel le dispositif de commande (4) détecte la taille et/ou le volume du manchon de compression (2), lorsque le manchon de compression (2) est raccordé au dispositif de gonflage (3), dans lequel le dispositif de commande (4) commande le dispositif de gonflage (3) pour le gonflage du manchon de compression (2) en utilisant l'ensemble de paramètres, lequel est associé à la taille et/ou au volume déterminé par le mécanisme de détection du manchon de compression (2) raccordé.

13. Dispositif de réalisation de garrot selon la revendication 12, **caractérisé en ce que** le mécanisme de détection détermine la taille et/ou le volume du manchon de compression (2) raccordé au moyen d'une mesure de pression ou au moyen d'une mesure de débit, dans lequel
- lors de la mesure de pression la pression dans le manchon de compression (2) raccordé est détectée, qui est générée lors du gonflage du manchon de compression au moyen du dispositif de gonflage (3) pendant une durée de détection (t1) prédéfinie, ou
- lors de la mesure de débit le débit d'un moyen de pression est détecté, qui est pompé lors du gonflage du manchon de compression (2) au moyen du dispositif de gonflage (3) pendant une durée de détection (t1) prédéfinie dans le manchon de compression (2) raccordé.
